(19) Europäisches Patentamt
European Patent Office
Office européen des brevets

(11) Veröffentlichungsnummer: **0 084 328**
**A2**

(12) # EUROPÄISCHE PATENTANMELDUNG

(21) Anmeldenummer: **83100083.1**

(22) Anmeldetag: **07.01.83**

(51) Int. Cl.³: **C 07 C 69/74**
C 07 C 61/04, C 07 C 67/08
C 07 C 51/00, A 01 N 53/00

(30) Priorität: **20.01.82 DE 3201481**

(43) Veröffentlichungstag der Anmeldung:
**27.07.83 Patentblatt 83/30**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Jautelat, Manfred, Dr.**
**Muellersbaum 28**
**D-5093 Burscheid(DE)**

(72) Erfinder: **Arlt, Dieter, Prof. Dr.**
**Rybniker Strasse 2**
**D-5000 Koeln 80(DE)**

(72) Erfinder: **Hagemann, Hermann, Dr.**
**Kandinskystrasse 52**
**D-5090 Leverkusen(DE)**

(72) Erfinder: **Hammann, Ingeborg, Dr.**
**Lutherstrasse 22**
**D-4330 Muelheim/Ruhr(DE)**

(72) Erfinder: **Homeyer, Bernhard, Dr.**
**Obere Strasse 28**
**D-5090 Leverkusen(DE)**

(54) **Halogenalkylsubstituierte Cyclopropancarbonsäureester, Verfahren zu ihrer Herstellung und ihre Verwendung als Schädlingsbekämpfungsmittel.**

(57) Die vorliegende Erfindung betrifft neue halogenalkylsubstituierte Cyclopropancarbonsäureester der allgemeinen Formel I

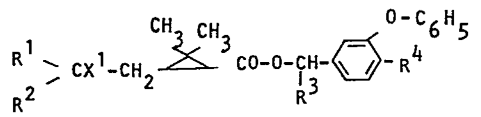
I,

in welcher
$R^1$, $R^2$, $R^3$, $R^4$, $X^1$ die in der Beschreibung angegebene Bedeutung haben.

Sie werden hergestellt, indem man Cyclopropancarbonsäuren der Formel II

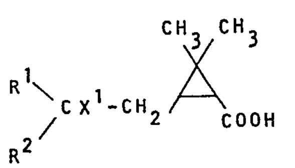
II

oder ihre reaktionsfähigen Derivate mit Alkoholen der Formel III

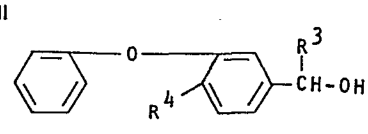
III .

oder deren reaktionsfähigen Derivaten umsetzt.

Sie eignen sich als Schädlingsbekämpfungsmittel.

EP 0 084 328 A2

BAYER AKTIENGESELLSCHAFT 5090 Leverkusen, Bayerwerk
Zentralbereich
Patente, Marken und Lizenzen Rt/bo/c
Ia

# Halogenalkylsubstituierte Cyclopropancarbonsäureester, Verfahren zu ihrer Herstellung und ihre Verwendung als Schädlingsbekämpfungsmittel

Die vorliegende Erfindung betrifft neue halogenalkylsubstituierte Cyclopropancarbonsäureester, Verfahren zu ihrer Herstellung, Zwischenprodukte dafür sowie ihre Verwendung in Schädlingsbekämpfungsmitteln.

Es ist bereits bekannt geworden, daß trichlorethylsubstituierte Cyclopropancarbonsäureester insektizide Wirksamkeit aufweisen (vgl. DE-OS 2 843 073). Ihre Wirkung befriedigt jedoch vor allem bei niedrigen Anwendungskonzentrationen in bestimmten Anwendungsbereichen nicht immer.

Die vorliegende Erfindung betrifft

1. Neue halogenalkylsubstituierte Cyclopropancarbonsäureester der allgemeinen Formel I

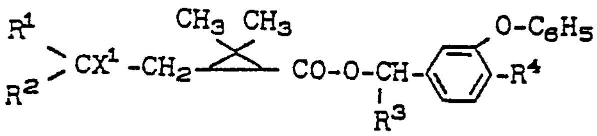

I,

in welcher

$R^1$ für Halogenalkyl das zusätzlich durch Alkoxy substituiert sein kann,

$R^2$ für Halogen oder Halogenalkyl,

$R^3$ für Wasserstoff, Cyano oder Ethynyl,

$R^4$ für Wasserstoff oder Fluor und

$X^1$ für Halogen steht;

2. ein Verfahren zur Herstellung der neuen halogenalkylsubstituierten Cyclopropancarbonsäureester der allgemeinen Formel I

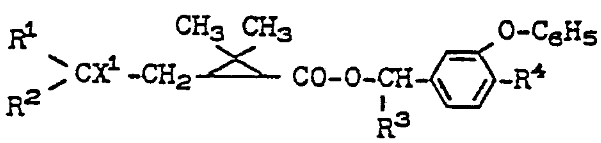

I,

in welcher

$R^1$ für Halogenalkyl,

$R^2$ für Halogen oder Halogenalkyl oder alkoxy-substituiertes Halogenalkyl,

$R^3$ für Wasserstoff, Cyano oder Ethynyl,

$R^4$ für Wasserstoff oder Fluor und

$X^1$ für Halogen steht;

das dadurch gekennzeichnet ist, daß man Cyclopropancarbonsäuren der Formel II

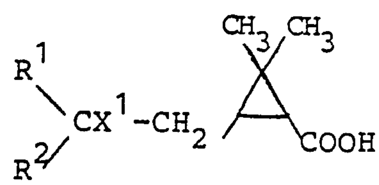

II

in welcher

$R^1$, $R^2$, $X^1$ die oben angegebene Bedeutung haben,

oder ihre reaktionsfähigen Derivate mit Alkoholen der Formel III

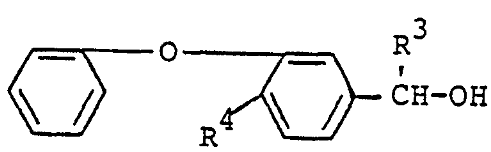

III

in welcher

$R^3$, $R^4$ die oben angegebene Bedeutung haben,

oder deren reaktionsfähigen Derivaten umsetzt;

3. Cyclopropancarbonsäuren der Formel II

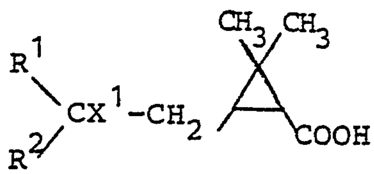

II

in welcher

$R^1$, $R^2$, $X^1$ die oben angegebene Bedeutung haben,

sowie deren Salze und Halogenide;

4. ein Verfahren zur Herstellung der Cyclopropancarbonsäuren der Formel II, das dadurch gekennzeichnet ist, daß man Ketone der Formel IV

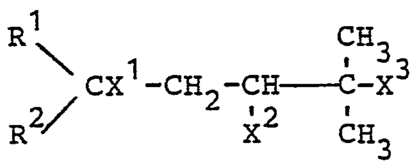

IV

in welcher

$R^1$, $R^2$, $X^1$ die oben angegebene Bedeutung haben und

$X^2$, $X^3$ für Cl, Br, J oder $-CO-CH_2-Cl$ stehen, wobei einer der Reste für $-CO-CH_2-Cl$ stehen muß,

mit Alkali oder Erdalkalihydroxiden bei Temperaturen zwischen 0 und 50°C umsetzt.

Die neuen substituierten Cyclopropancarbonsäureester weisen vor allem gute insektizide und akarizide Eigenschaften auf.

Sie sind durch Formel I allgemein definiert. In Formel I stehen vorzugsweise

$R^1$ für $C_{1-4}$-Halogenalkyl mit 3 - 9 Halogenatomen, insbesondere Fluor, Chlor, Brom, das zusätzlich durch $C_{1-4}$-Alkoxy substituiert sein kann;

$R^2$ für Fluor, Chlor, Brom, $C_{1-4}$-Halogenalkyl mit 3 - 9 Halogenatomen;

$R^3$ für Wasserstoff, Cyano oder Ethinyl;

$R^4$ für Wasserstoff oder Fluor;

$X^1$ für Fluor, Chlor, Brom.

Besonders bevorzugt sind Verbindungen der Formel I in welcher

$R^1$ für Trifluormethyl, Chlordifluormethyl, Pentafluorethyl, Methoxydifluormethyl steht,

$R^2$ für Fluor, Chlor, Brom oder Trifluormethyl steht,

$R^3$ für Wasserstoff oder Cyano steht,

$R^4$ für Wasserstoff oder Fluor steht,

$X^1$ für Fluor, Chlor, Brom steht.

Insbesondere seien genannt Verbindungen der Formel I in welcher

$R^1$ für Fluormethyl,
$R^2$ für Chlor,
$R^3$ für Wasserstoff oder Cyano,
$R^4$ für Wasserstoff oder Fluor,
$X^1$ für Chlor steht.

Im Einzelnen seien genannt:

2,2-Dimethyl-3-(2,2-dichlor-3,3,3-trifluorpropyl)-cyclopropancarbonsäure-(α-cyano-3-phenoxybenzyl)-ester
2,2-Dimethyl-3-(2,2-dichlor-3,3,3-trifluorpropyl)-cyclopropancarbonsäure-(α-cyano-4-fluor-3-phenoxybenzyl)-ester
2,2-Dimethyl-3-(2,3,3,3-tetrafluor-2-trifluormethylpropyl)-cyclopropancarbonsäure-(α-cyano-3-phenoxybenzyl)-ester
2,2-Dimethyl-3-(2,3,3,3-tetrafluor-2-trifluormethylpropyl)-cyclopropansäure-(α-cyano-4-fluor-3-phenoxybenzyl)-ester
2,2-Dimethyl-3-(2,2,3-trichlor-3,3-difluorpropyl)-cyclopropancarbonsäure-(α-cyano-3-phenoxy)-ester
2,2-Dimethyl-3-(2,2,3-trichlor-3,3-difluorpropyl)-cyclopropancarbonsäure-(α-cyano-4-fluor-3-phenoxybenzyl)-ester

2,2-Dimethyl-3-(2,2-dichlor-3,3,4,4,4-pentafluorbutyl)-cyclopropancarbonsäure-(α-cyano-3-phenoxybenzyl)-ester

2,2-Dimethyl-3-(2,2-dichlor-3,3,4,4,4-pentafluorbutyl)-cyclopropancarbonsäure-(α-cyano-4-fluor-3-phenoxybenzyl)-ester

2,2-Dimethyl-3-(2,2-dibrom-3,3,3-trifluorpropyl)-cyclopropancarbonsäure-(α-cyano-3-phenoxybenzyl)-ester

2,2-Dimethyl-3-(2,2-dibrom-3,3,3-trifluorpropyl)-cyclopropancarbonsäure-(α-cyano-4-fluor-3-phenoxybenzyl)-ester

2,2-Dimethyl-3-(2,2-dibrom-3,3-difluor-3-methoxypropyl)-cyclopropancarbonsäure-(α-cyano-3-phenoxybenzyl)-ester

2,2-Dimethyl-3-(2,2-dibrom-3,3-difluor-3-methoxypropyl)-cyclopropancarbonsäure-(α-cyano-4-fluor-3-phenoxybenzyl)-ester

Eine bevorzugte Variante (a) des unter (2) dargelegten Verfahrens zur Herstellung der neuen Verbindungen der Formel (I) - 'Verfahren (2 a)' - ist dadurch gekennzeichnet, daß man als reaktionsfähige Derivate der halogenalkylsubstituierten Cyclopropancarbonsäuren der Formel (II) Cyclopropancarbonsäurechloride der Formel IIa

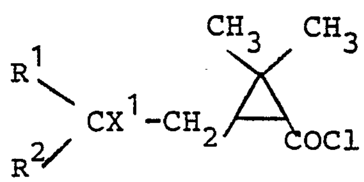

(IIa)

in welcher

$X^1$, $R^1$ und $R^2$ die unter (1) angegebenen Bedeutungen haben,

mit den Alkoholen der Formel (III) (oben) in Gegenwart von Säureakzeptoren und unter Verwendung von Verdünnungsmitteln umsetzt.

Eine weitere bevorzugte Variante (b) des unter (2) dargelegten Verfahrens zur Herstellung von Verbindungen der Formel (I), in welcher $R^3$ für Cyano steht - 'Verfahren (2 b)' - ist dadurch gekennzeichnet, daß man Carbonsäurechloride der Formel IIa (oben) mit Aldehyden der Formel IIIa

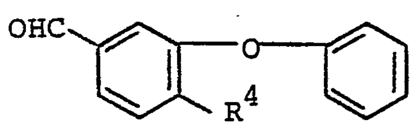

. (IIIa)

in welcher

$R^4$ die unter (1) angegebene Bedeutung hat,

und wenigstens der äquimolaren Menge eines wasserlöslichen Cyanids (vorzugsweise Natriumcyanid oder Kaliumcyanid), gegebenenfalls in Gegenwart eines Katalysators und unter Verwendung von Verdünnungsmitteln umsetzt.

Verwendet man als Ausgangsstoffe beispielsweise 2,2-Dimethyl-3-(2,2-dichlor-3,3,3-trifluorpropyl)-cyclopropancarbonsäurechlorid und 3-Phenoxybenzylalkohol bzw.

3-Phenoxy-4-fluorbenzaldehyd und Natriumcyanid, so können die beiden Verfahrensvarianten durch folgendes Formelschema skizziert werden:

(2 a)

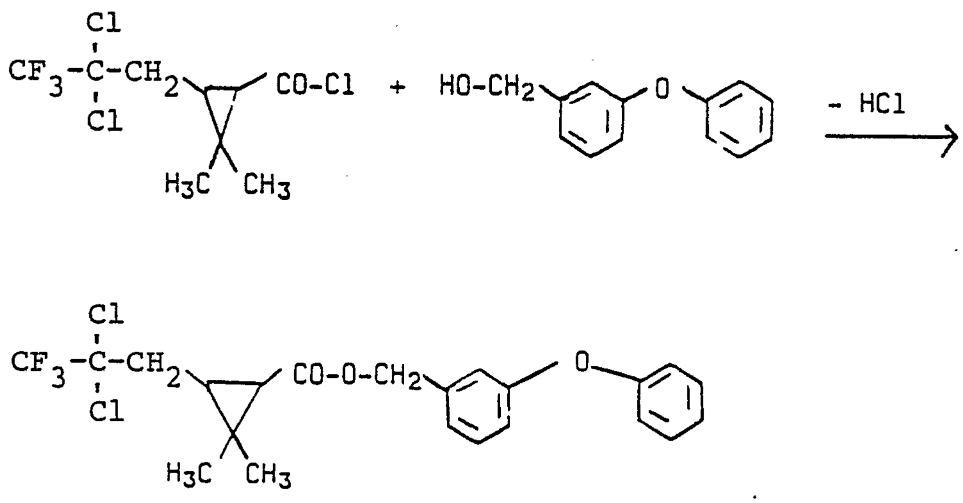


(2 b)

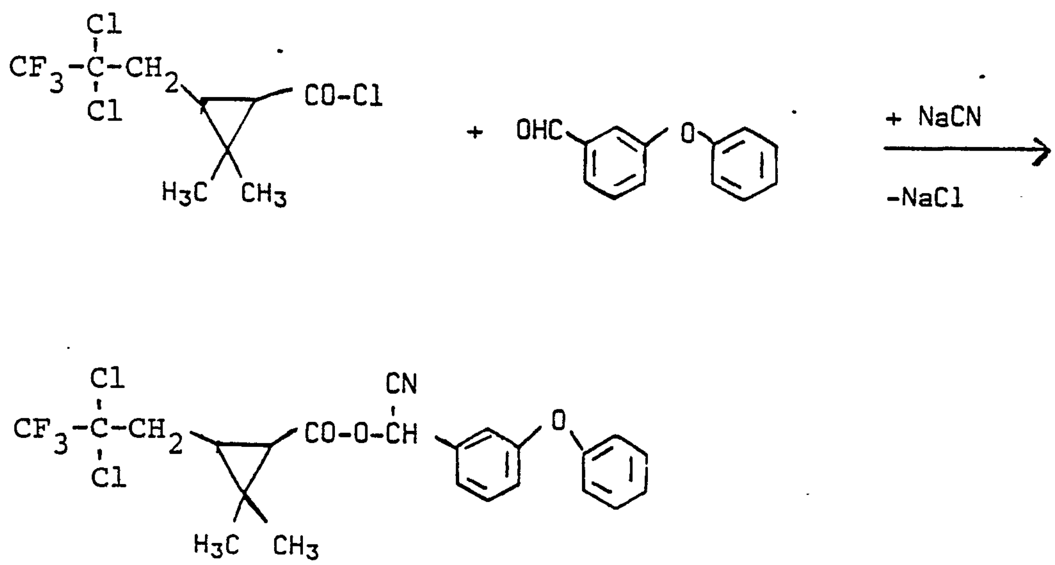

Die verschiedenen Varianten von Verfahren (2) werden im allgemeinen unter Verwendung von Verdünnungsmitteln durchgeführt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methylisopropyl- und Methylisobutylketon, Ester wie Essigsäuremethylester und -ethylester, Nitrile, wie z.B. Acetonitril und Propionitril. Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Verfahrensvariante (2 b) wird vorzugsweise unter Verwendung von Wasser als zweiter Solvenskomponente neben einem mit Wasser nicht mischbaren organischen Lösungsmittel aus der Reihe der Kohlenwasserstoffe und gegebenenfalls in Gegenwart eines Katalysators durchgeführt. Als Katalysatoren werden gegebenenfalls Verbindungen verwendet, welche zum Transfer von Anionen aus Wasser in organische Lösungsmittel geeignet sind. Beispiele hierfür sind Benzyltriethyl-ammonium-hydrogensulfat, Tetrabutylammonium-bromid und Methyl-tricaprylammoniumchlorid (Aliquat 336).

Bei der Verfahrensvariante (2 a) können die üblichen Säurebindemittel verwendet werden. Als Beispiele seien genannt: Alkalihydroxide, wie z.B. Natrium- und Kaliumhydroxid, Alkalicarbonate, wie z.B. Natrium- und Kaliumcarbonat, Alkalialkoholate, wie z.B. Natrium- und Kaliummethylat und -ethylat, ferner aliphatische und aromatische, auch heterocyclische Amine, wie z.B. Triethyl- und Trimethylamin, N,N-Dimethyl-anilin, N,N-Dimethylbenzylamin, Pyridin, Diazabicyclooctan, Diazabicyclononen und Diazabicycloundecen.

Die Reaktionstemperatur wird bei Verfahren (2) zwischen 0 und 100°C, bei den Varianten (a) und (b) vorzugsweise zwischen 10 und 50°C gehalten. Das Verfahren wird im allgemeinen bei Normaldruck durchgeführt.

Zur Durchführung von Verfahren (2) - Varianten (a) und (b) - werden angenähert äquimolare Mengen der Ausgangsstoffe, gegebenenfalls zusammen mit Säureakzeptoren bzw. Katalysatoren, in geeigneten Verdünnungsmitteln zusammengegeben und die Reaktionsgemische werden mehrere Stunden gerührt. Zur Aufarbeitung, welche nach üblichen Methoden erfolgen kann, wird gegebenenfalls mit Wasser und/oder einem mit Wasser nicht mischbaren organischen Lösungsmittel verdünnt, die organische Phase abgetrennt, mit Wasser gewaschen, getrocknet und filtriert. Das Lösungsmittel wird unter vermindertem Druck sorgfältig abdestilliert, wobei das Rohprodukt als Rückstand verbleibt.

Die als Ausgangsstoffe zu verwendenden neuen Cyclopropancarbonsäuren sind durch die Formel (II), die entsprechenden Säurechloride durch die Formel (IIa) definiert. Vorzugsweise haben in diesen Formeln $R^1$ und $R^2$ die gleichen Bedeutungen, wie sie bei der Definition der entsprechenden Gruppen in Formel (I) als bevorzugt angegeben sind.

Die Carbonsäurechloride der Formel (IIa) erhält man aus den entsprechenden Carbonsäuren der Formel (II) nach üblichen Methoden, beispielsweise durch Umsetzung mit Thionylchlorid, gegebenenfalls unter Verwendung eines Verdünnungsmittels, wie z.B. Tetrachlorkohlenstoff bei Temperaturen zwischen 0 und 100°C und anschließende Destillation.

Die Cyclopropancarbonsäuren der Formel II sind neu. In Formel II haben die Reste $R^1$, $R^2$ und $X^1$ die bei den entsprechenden Substituenten der Verbindungen der Formel I angegebenen vorzugsweisen Bedeutungen.

Die Cyclopropancarbonsäuren der Formel II werden nach dem unter 4) (oben) angegebenen Verfahren hergestellt. Danach werden Ketone der Formel IV als Gemisch oder nach einer Trennung einzeln mit Alkalihydroxiden oder Erdalkalihydroxiden in Gegenwart eines Verdünnungsmittels umgesetzt.

Beispielhaft wird die Reaktion durch das folgende Formelschema wiedergegeben:

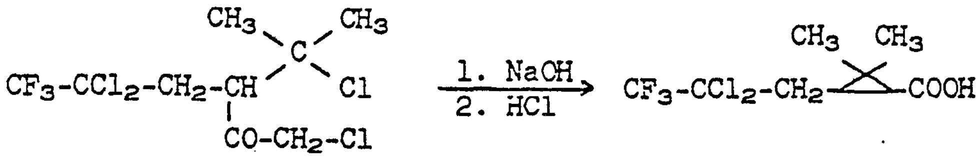


Die Umsetzung mit Alkali- und Erdalkalihydroxiden, vorzugsweise Natriumhydroxid und Kaliumhydroxid, erfolgt bevorzugt in wäßriger oder alkoholischer Lösung bei Temperaturen von 0° bis 50°, vorzugsweise 15° bis 30°.

Zur Durchführung des erfindungsgemäßen Verfahrens setzt man 1 Mol der Ketone der Formel IV mit 2 bis 5 Äquivalenten an Hydroxiden um. Auch ein größerer Überschuß an Base ist möglich. Die Säure wird aus der Reaktionsmischung nach Ansäuern isoliert.

Es ist als ausgesprochen überraschend zu bezeichnen, daß die Einwirkung von Hydroxiden auf die Ketone der Formel II und/oder III bei Temperaturen unterhalb von 50° zu gesättigten Cyclopropancarbonsäuren der Formel IV führt, während die Umsetzung mit Alkoholaten und Hydroxiden bei Temperaturen oberhalb von 50° Vinylcyclopropancarbonsäure-Derivate ergibt.

Die Ketone der Formel IV werden analog zu einem noch nicht zum Stand der Technik gehörenden Verfahren, das Gegenstand einer älteren Anmeldung der Anmelderin ist, erhalten (DE-Anm. P 31 00 354.0). Dabei werden Halogenalkane der Formel

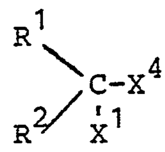

V

in welcher

$R^1$, $R^2$, $X^1$ die unter 1) (oben) angegebene Bedeutung haben und

$X^4$ für Halogen steht

mit 1-Chlor-3,3-dimethyl-pent-4-en-2-on in Gegenwart von Katalysatoren, die freie Radikale liefern oder in Gegenwart von Metallsalzen der VIII. Hauptgruppe und der Nebengruppen IVa, VIIa und Ib des Periodensystems addiert.

Verwendet man beispielsweise 1,1,1-Trichlor-2,2,2-trifluorethan als Ausgangsstoff, so kann der Reaktionsablauf durch das folgende Formelschema wiedergegeben werden:

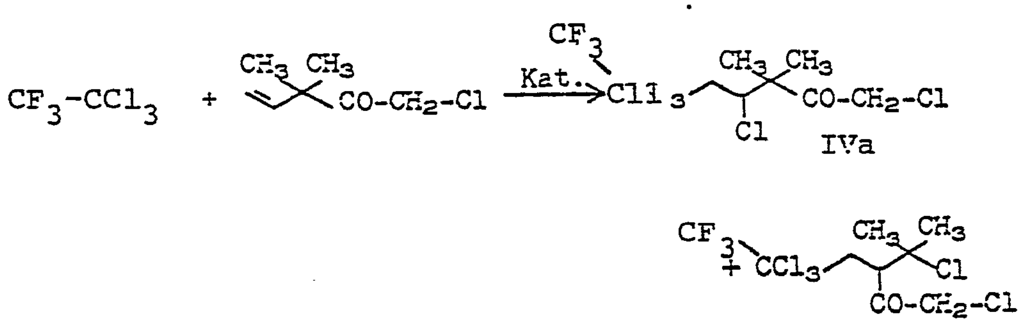


IVa

IV b

Die Halogenalkane als Ausgangsstoffe sind durch die Formel V allgemein definiert. In der Formel V steht $X^1$ vorzugsweise für Chlor, Brom, Fluor, $R^1$ bzw. $R^2$ für Chlor, Fluor oder perfluorierte Alkylreste mit 1 - 3 C-Atomen. $X^4$ steht vorzugsweise für Chlor oder Brom.

Als Beispiele für die erfindungsgemäßen verwendbaren Halogenalkane seien im einzelnen genannt: 1,1,1-Trichlortrifluorethan, 1,1,1-Tribromtrifluorethan, 1,1-Dibromtetrafluorethan, 2,2-Dibrom-1,1,1,3,3,3-hexafluorpropan, 1,1,1,3-Tetrachlortetrafluorpropan, 1,1,1-Trichlorpentafluorpropan.

1-Chlor-3,3-dimethyl-pent-4-en-2-on kann in einfacher Weise nach folgendem Verfahren hergestellt werden.

Die Chlorwasserstoffaddition an Isopren führt zum Dichlorpentan, das an Vinylidenchlorid addiert und mit Basen zum 1,1-Dichlor-3,3-dimethyl-1,4-pentadien eliminiert wird. Umsetzung mit Natriumphenolat und Hydrolyse liefern das Chlormethylketon der Formel VI:

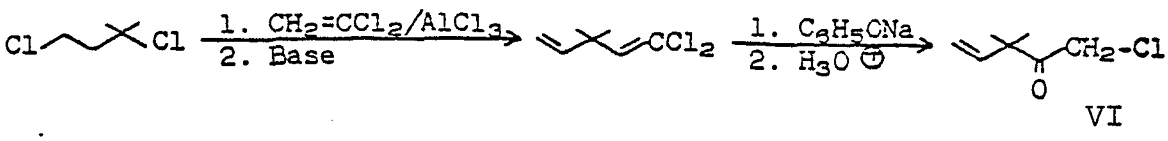


VI

Die Addition der Polyhalogenalkane der Formel V an das 1-Chlor-3,3-dimethyl-pent-4-en-2-on wird bei Normaldruck, im Falle von niedersiedenden Polyhalogenalkanen der Formel V vorzugsweise unter Druck durchgeführt.

Der Druckbereich kann in weiten Grenzen schwanken, etwa zwischen 1 und 30 bar, bevorzugt zwischen 3 und 15 bar. Der Überdruck kann durch Aufpressen eines Inertgases, wie beispielsweise Stickstoff, erreicht werden.

Die Umsetzungstemperatur kann zwischen 50 und 200°C liegen, bevorzugt wird jedoch zwischen 80 und 150°C gearbeitet. Den beiden Ausgangsstoffen der Formeln V und VI wird gegebenenfalls ein Verdünnungsmittel zugesetzt.

Als Verdünnungsmittel kommen in Frage: aliphatische oder aromatische Kohlenwasserstoff, wie Benzin oder Toluol, Alkohole, wie Methanol, Ethanol, Propanol oder tert.-Butanol, Nitrile wie Acetonitril oder Propionitril, DMF. Bevorzugt sind Alkohole, Acetonitril und DMF.

Zur Durchführung der Reaktion sind Katalysatoren erforderlich. Und zwar entweder

a) freie Radikale liefernde Katalysatoren, wie z.B. Azobisisobutyronitril, Benzoylperoxid oder Di-tert.-butylperoxid oder

b) Metallsalze der VIII. Hauptgruppe und der Nebengruppe VI a, VII a und I b des periodischen Systems. Als Beispiele seien genannt: Kupfer(II)oxid, Eisen-(II)oxid; Cu(I)-, Cu(II)-, Fe(II)- und Fe(III)bromide und vor allem -chloride sowie die Chloride des Rutheniums, Rhodiums, Palladiums, Kobalts und Nickels; Cu(II)sulfat, Fe(II)- und Fe(III)sulfat; Cu-(II)-nitrat und Eisen(III)nitrat; Mangan(III)acetat, Kupfer(II)acetat; Kupfer(II)stearat; Eisen-(III)citrat; Cu(I)cyanid; Rhuthenium(II)dichlor-tris-triphenylphosphin, Rhodium-tris-(triphenylphosphin)-chlorid; Chrom- und Nickelacetylacetonat, Kupfer(II)acetylacetonat, Eisen(III)acetylacetonat, Kobalt(II)- und Kobalt(III)acetylacetonat, Mangan-(II)acetylacetonat, Kupfer(II)benzoylacetonat;

Eisencarbonyl-cyclopentadienyl-komplex; Molybdäncarbonylcyclopentadienylkomplex, Rhuthenium(II)acetatkomplex, Chrom- und Molybdänhexacarbonyl, Nickeltetracarbonyl, Eisenpentacarbonyl, Kobalt- und Mangancarbonyl oder

c) Katalysatormischungen, die aus einem der obigen Metallsalze, einem Amin und gegebenenfalls etwas Benzoin bestehen. Das Metallsalz kann auch als Hydrat vorliegen, das Amin kann auch als Salz, z.B. als Hydrochlorid vorliegen. Beispiele für Amine sind: Dimethylamin, Diethylamin, Trimethylamin, Triethylamin, Diisopropylamin, n-Butylamin, Benzylamin, Ethanolamin, Anilin, Pyridin. Bevorzugt sind Dimethylamin, Diethylamin (als Hydrochloride), sowie n-Butylamin.

Es werden vorzugsweise mindestens 1,5 Mol, bevorzugt 2 bis 10 Mol organisches Amin pro Mol Metallsalz eingesetzt. Das Metallsalz wird in Mengen von 0,01 bis 15 Mol-%, vorzugsweise 0,05 bis 10 Mol-%, bezogen auf Verbindungen der Formel VI, eingesetzt.

Die Zugabe von äquimolaren Mengen Benzoin bezogen auf das Metallsalz ist oft von Vorteil.

Das Polyhalogenalkan (V) wird vorzugsweise im Überschuß eingesetzt oder gar als Verdünnungsmittel verwendet.

Die Ketone der Formel IV fallen dabei als Gemisch der Isomeren der Formel IV a und IV b (oben) an.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo - und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.



BAD ORIGINAL

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele:

Beispiel 1

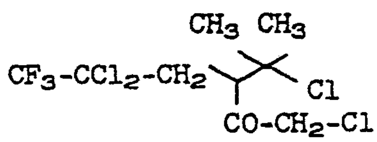

24,3 g (0,16 Mol) 1-Chlor-3,3-dimethyl-4-penten-2-on und 62,1 g (0,33 Mol) 1,1,1-Trichlor-2,2,2-trifluorethan werden mit 0,2 g Eisen-(III)-chloridhydrat, 0,16 g Benzoin, 0,06 g Dimethylamin-hydrochlorid, 1,25 g Eisenspänen sowie 13,6 g Acetonitril 7 Std. in einem Bombenrohr auf 120° erhitzt. Das Reaktionsgemisch wird filtriert. Durch Destillation ($Kp_{0,08}$ 89°) isoliert man 75 % 1,5,5-Trichlor-6,6,6-trifluor-3-(2-chlor-propyl-2)-hexan-2-on.

Beispiel 2

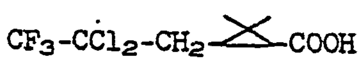

35,5 g (0,1 Mol) 1,5,5-Trichlor-6,6,6-trifluor-3-(2-chlor-propyl-2)-hexan-2-on werden mit 16 g (0,4 Mol) Natriumhydroxid in 200 ml Wasser 7 Std. bei 20° gerührt. Die klare Lösung wird durch Ausschütteln mit Methylenchlorid, das verworfen wird, gereinigt. Dann wird mit Salzsäure angesäuert und mit Methylenchlorid mehrfach extrahiert. Nach dem Trocknen der Lösung über $Na_2SO_4$ wird das Lösungsmittel im Vakuum abgezogen. Es bleiben 25,5 g (0,092 Mol, 92 %) 2,2-Dimethyl-3-(2,2-dichlor-3,3-trifluorpropyl)-cyclopropancarbonsäure als cistrans-Gemisch vom Fp. 100 - 120° zurück.

Beispiel 3

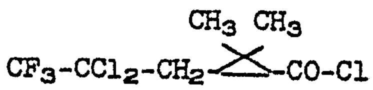

Zu 86 g (0,31 Mol) 2,2-Dimethyl-3-(2,2-dichlor-3,3,3-trifluorpropyl)-cyclopropancarbonsäure und einem Tropfen Dimethylformamid tropft man bei 40° 40 g (0,34 Mol) Thionylchlorid. Dann werden 90 Min. auf 80° erwärmt. Die direkte Destillation liefert bei $Kp_{0,1}$ 68 - 85° 77 g (0,26 Mol, 84 %) 2,2-Dimethyl-3-(2,2-dichlor-3,3,3-trifluorpropyl)-cyclopropancarbonsäurechlorid als cis-trans-Gemisch.

NMR ($CDCl_3$): δ 1,25 - 1,35 (3 Linien, 6 H), 1,85 - 2,7 (m, 4 H)

Beispiel 4

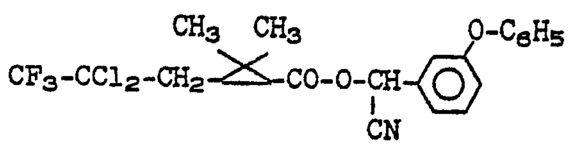

32,5 g (0,5 Mol) Kaliumcyanid und 2 g Tetrabutylammoniumbromid werden in 500 ml Wasser vorgelegt. Dann tropft man die Lösung von 76 g (0,25 Mol) 2,2-Dimethyl-3-(2,2-dichlor-3,3,3-trifluorpropyl)-cyclopropancarbonsäurechlorid und 46 g (0,23 Mol) 3-Phenoxybenzaldehyd in 500 ml Toluol zu und rührt 4 Std. bei R. T. nach. Das Reaktionsgemisch wird alkalisch gestellt und mehrfach mit Toluol ausgeschüttelt. Nach Trocknen der Toluollösung wird das Toluol im Hochvakuum abgezogen. Es

bleiben 112 g (0,23 Mol, 100 %) 2,2-Dimethyl-3-(2,2-dichlor-3,3-trifluorpropyl)-cyclopropancarbonsäure-(α-cyano-3-phenoxy-benzyl)-ester als Öl zurück. Das Isomerenverhältnis cis:trans beträgt 40:60.

NMR ($CDCl_3$): δ

cis-Ester 1,2 (2 s, 6 H), 1,5 - 1,9 (m 2 H), 2,65 (m, 2 H), 6,3 (s, 1 H), 6,9 - 7,5 (m, 8 H)

trans-Ester 1,2 (2 s, 6H), 1,4 - 2,05 (m, 2H) 2,4 (m, 2 H), 6,35 (s, 1 H), 6,85 - 7,5 (m, 8 H)

Beispiel 5

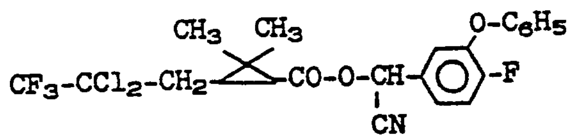

7,15 g (0,11 Mol) Kaliumcyanid und 0,44 g Tetrabutylammoniumbromid werden in 50 ml Wasser gelöst. Bei Raumtemperatur tropft man die Lösung von 16,6 g (55 mMol) 2,2-Dimethyl-3-(2,2-dichlor-3,3,3-trifluorpropyl)-cyclopropancarbonsäurechlorid und 11,2 g (52 mMol) 4-Fluor-3-phenoxy-benzaldehyd in 55 ml Toluol zu und rührt 3 Std. nach. Die Aufarbeitung analog Beispiel 4 ergibt 26,2 g (52 mMol, 100 %) 2,2-Dimethyl-3-(2,2-dichlor-3,3,3-trifluorpropyl)-cyclopropancarbonsäure-(α-cyano-4-fluor-3-phenoxybenzyl)-ester als Öl.

0084328

Beispiel A

Laphygma-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 4, 5.

Beispiel B

Tetranychus-Test (resistent)

| | | |
|---|---|---|
| Lösungsmittel: | 3 Gewichtsteile | Aceton |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 4, 5.

Beispiel C

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 4, 5.

Patentansprüche

1. Halogenalkylsubstituierte Cyclopropancarbonsäureester der allgemeinen Formel I

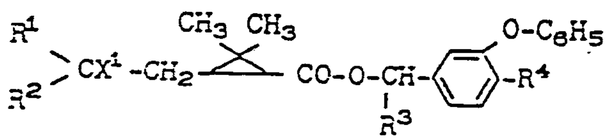

I,

in welcher

$R^1$ für Halogenalkyl das zusätzlich durch Alkoxy substituiert sein kann,

$R^2$ für Halogen oder Halogenalkyl,

$R^3$ für Wasserstoff, Cyano oder Ethynyl,

$R^4$ für Wasserstoff oder Fluor und

$X^1$ für Halogen steht.

2. Verfahren zur Herstellung der halogenalkylsubstituierten Cyclopropancarbonsäureester der allgemeinen Formel I

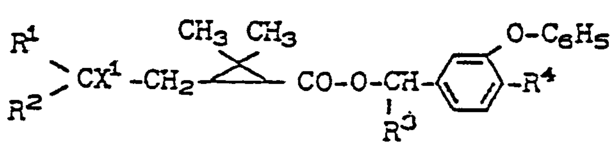

I,

in welcher

$R^1$ für Halogenalkyl,

$R^2$ für Halogen oder Halogenalkyl oder alkoxysubstituiertes Halogenalkyl,

$R^3$ für Wasserstoff, Cyano oder Ethynyl,

$R^4$ für Wasserstoff oder Fluor und

$X^1$ für Halogen steht;

das dadurch gekennzeichnet ist, daß man Cyclopropancarbonsäuren der Formel II

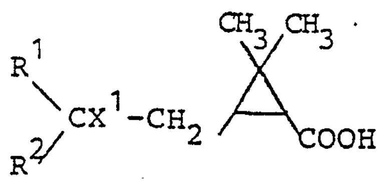

II

in welcher

$R^1$, $R^2$, $X^1$ die oben angegebene Bedeutung haben,

oder ihre reaktionsfähigen Derivate mit Alkoholen der Formel III

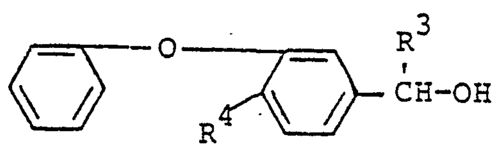

III

in welcher

$R^3$, $R^4$ die oben angegebene Bedeutung haben

oder deren reaktionsfähigen Derivaten umsetzt.

3. Cyclopropancarbonsäuren der Formel II

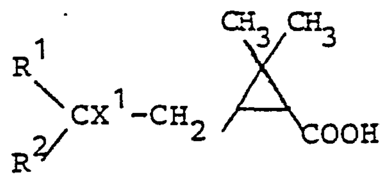

II

in welcher

$R^1$, $R^2$, $X^1$ die in Anspruch 1 angegebene Bedeutung haben

sowie deren Salze und Halogenide.

4. Verfahren zur Herstellung der Cyclopropancarbonsäuren der Formel II gemäß Anspruch 3, das dadurch gekennzeichnet ist, daß man Ketone der Formel IV

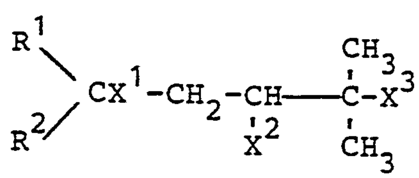

IV

in welcher

$R^1$, $R^2$, $X^1$ die oben angegebene Bedeutung haben und

$X^2$, $X^3$ für Cl, Br, J oder $-CO-CH_2-Cl$ stehen, wobei einer der Reste für $-CO-CH_2-Cl$ stehen muß,

mit Alkali oder Erdalkalihydroxiden bei Temperaturen zwischen 0 und 50°C umsetzt.

5. Cyclopropancarbonsäureester der Formel I, in welcher

$R^1$ für $C_{1-4}$-Halogenalkyl mit 3 - 9 Halogenatomen, insbesondere Fluor, Chlor, Brom, das zusätzlich durch $C_{1-4}$-Alkoxy substituiert sein kann steht,

$R^2$ für Fluor, Chlor, Brom, $C_{1-4}$-Halogenalkyl mit 3 - 9 Halogenatomen steht,

$R^3$ für Wasserstoff, Cyano oder Ethinyl steht,

$R^4$ für Wasserstoff oder Fluor steht,

$X^1$ für Fluor, Chlor, Brom steht.

6. Schädlingsbekämpfungsmittel, gekennzeichnet durch einen Gehalt an mindestens einem halogenalkylsubstituierten Cyclopropancarbonsäureester der Formel (I).

7. Verwendung von halogenalkylsubstituiertem Cyclopropancarbonsäureester der Formel (I) zur Bekämpfung von Schädlingen.

8. Verfahren zur Bekämpfung von Schädlingen, dadurch gekennzeichnet, daß man halogenalkylsubstituierte Cyclopropancarbonsäureester der Formel (I) auf Schädlinge und/oder ihren Lebensraum einwirken läßt.

9. Verfahren zur Herstellung von Schädlingsbekämpfungsmitteln, dadurch gekennzeichnet, daß man halogenalkylsubstituierte Cyclopropancarbonsäureester der Formel (I) mit Streckmitteln und/oder oberflächenaktiven Mitteln vermischt.